# EUROPEAN PATENT SPECIFICATION

(11) **EP 1 188 445 B1**
(45) Date of publication and mention of the grant of the patent: **21.11.2007**
(21) Application number: 00939144.2
(22) Date of filing: 23.06.2000
(51) Int. Cl.: A61K 38/44, A61K 47/26, A61K 9/19, A61P 43/00, A61P 1/04, A61P 25/00

(54) **DRUG COMPOSITION CONTAINING LECITHIN-MODIFIED SUPEROXIDE DISMUTASE**
MEDIKAMENTZUSAMMENSETZUNG DIE LECITHIN-MODIFIZIERTE SUPEROXIDDISMUTASE ENTHÄLT
COMPOSITION MEDICAMENTEUSE CONTENANT UNE SUPEROXYDE DISMUTASE MODIFIEE PAR LECITHINE

(30) Priority: 24.06.1999 JP 17822799
(43) Date of publication of application: 20.03.2002
(73) Proprietor: LTT Bio-Pharma Co., Ltd., Tokyo 105-6201 (JP)
(72) Inventor: IKEDA, Yoshihito, Higashiyamato-shi, Tokyo 207-0021 (JP); SATOU, Hiromi, Musashimurayama-shi, Tokyo 208-0013 (JP)
(74) Representative: Rupp, Christian
(86) International application number: PCT/JP2000/004138
(87) International publication number: WO 2001/000230

(56) References cited:
- EP-A- 0 355 348
- JP-A- 1 304 882
- JP-A- 6 054 681
- JP-A- 9 117 279
- US-A- 5 534 251
- US-A- 5 762 929
- IKEDA K ET AL: "Lecithinized superoxide dismutase retards wobbler mouse motoneuron disease." NEUROMUSCULAR DISORDERS: NMD. ENGLAND SEP 1995, vol. 5, no. 5, September 1995 (1995-09), pages 383-390, XP002278396 ISSN: 0960-8966
- DATABASE WPI Section Ch, Week 199718 Derwent Publications Ltd., London, GB; Class B04, AN 1997-197216 XP002278397 & JP 09 052843 A (SAMU KENKYUSHO KK), 25 February 1997 (1997-02-25)
- HORI YUSUKE ET AL: "Effect of lecithinized-superoxide dismutase on the rat colitis model induced by dextran sulfate sodium" JAPANESE JOURNAL OF PHARMACOLOGY, vol. 74, no. 1, 1997, pages 99-103, XP009030058 ISSN: 0021-5198
- DATABASE WPI Section Ch, Week 199251 Derwent Publications Ltd., London, GB; Class B04, AN 1992-418587 XP002278398 & JP 04 312533 A (NIPPON KAYAKU KK), 4 November 1992 (1992-11-04)

### SEQUENCE LISTING

<110> LTT Institute Co.,Ltd., and Seikagaku Corporation
<120> Pharmaceutical composition containing lecithinized-superoxide dismutase
<130>
<160> 1
<210> 1
<211> 153
<212> PRT
<213> Homo sapiens
<400> 1
   Ala Thr Lys Ala Val Cys Val Leu Lys Gly Asp Gly Pro Val Gln Gly
   1 5 10 15
   Ile Ile Asn Phe Glu Gln Lys Glu Ser Asn Gly Pro Val Lys Val Trp
   20 25 30
   Gly Ser Ile Lys Gly Leu Thr Glu Gly Leu His Gly Phe His Val His
   35 40 45
   Glu Phe Gly Asp Asn Thr Ala Gly Cys Thr Ser Ala Gly Pro His Phe
   50 55 60
   Asn Pro Leu Ser Arg Lys His Gly Gly Pro Lys Asp Glu Glu Arg His
   65 70 75 80
   Val Gly Asp Leu Gly Asn Val Thr Ala Asp Lys Asp Gly Val Ala Asp
   85 90 95
   Val Ser Ile Glu Asp Ser Val Ile Ser Leu Ser Gly Asp His Cys Ile
   100 105 110
   Ile Gly Arg Thr Leu Val Val His Glu Lys Ala Asp Asp Leu Gly Lys
   115 120 125
   Gly Gly Asn Glu Glu Ser Thr Lys Thr Gly Asn Ala Gly Ser Arg Leu
   130 135 140
   Ala Cys Gly Val Ile Gly Ile Ala Gln
   145 150

## Description

### Technical Field

The present invention relates to a lyophilized drug composition containing a lecithin-modified superoxide dismutase (hereinafter, sometimes referred to as simply PC-SOD) and sucrose as a drug carrier and to a lyophilized drug composition composed of them for use in the treatment of motor neuron disease or ulcerative gastrointestinal injury. The present invention also relates to a method for producing said lyophilized drug composition using sucrose as an effective ingredient.

### Background Art

Prior arts closest to the present invention will be described.
(a) PC-SOD usable in the present invention and a drug containing this as an active ingredient are described in Japanese Patent Laid-Open No. 9-117279.
(b) A biologically active protein composition for oral and topical administrations containing a lecithin-modified biologically active protein is described in Japanese Patent Laid-Open No. 3-170438. Superoxide dismutase (hereinafter, sometimes referred to as simply SOD) is described as an example of the biologically active protein and sugar is exemplified as an additive.

lkeda K. et al. (Neuromuscular disorders: NMD. England SEP 1995, vol. 5, no. 5, pages 383 to 390) disclose a lecithinized superoxide dismutase (PC-SOD) of Cu/Zn-type. The presented experimental results raise the possibility that PC-SOD have therapeutic potential in human motoneuron disease.

US-A-5 762 929 relates to a novel treatment method for motoneuron disease by administrating a therapeutic agent comprising superoxide dismutase conjugated with lecithin through chemical crosslinking. It discloses the use of mannitol as a drug carrier.

XP-002278397 (Database WPI Section, 1997) is directed to a renal insufficiency treating agent comprising a lecithin-modified SOD.

A drug composition characterized by including mannitol and a lecithin-modified superoxide dismutase has been described by Hori Yusuke et al. (Japanese Journal of Pharmacology, vol. 74, no. 1, 1997, pages 99-103).

JP-A-9117279 and JP-A-6054681 relate to a lecithinized superoxide dismutase. The medicament described therein comprises the lecithinized superoxide dismutase as an active ingredient. PC-SOD is obtained by bonding lysolecithin by chemical crosslinking to a superoxide dismutase (SOD).

However, in these prior arts, any specific combination for a drug composition containing PC-SOD and sucrose is not described. In addition, there were no solutions for problems including: a reduction of activity of PC-SOD due to long term storage; property when lyophilized; and appearances of peaks of analogues when analyzed by column chromatography.

### Disclosure of the Invention

An object of the present invention is to provide a drug composition containing PC-SOD with a less reduction of PC-SOD activity (i.e. higher stability) due to long term storage; good property when lyophilized; and controlled appearances of peaks of analogues when analyzed by column chromatography.

The present inventors examined assiduously about a drug composition having PC-SOD as an active ingredient in order to solve the above described problems, and consequently found that : sucrose as a drug carrier inhibits a reduction of PC-SOD activity due to long term storage (maintains stability); retains good property when lyophilized; has an action of controlling appearances of peaks of analogues when analyzed by column chromatography; and that this enables to provide a drug composition which can solve the above problems, leading completion of the present invention.

That is, the present invention provides a lyophilized drug composition (hereinafter, referred to as the drug composition of the present invention) characterized by including PC-SOD represented by the following general formula (I) and sucrose as a drug carrier:

SOD' (Q - B)ₘ (I)

characterized in that SOD' is a residue of superoxide dismutase; Q is a chemical crosslinking; B is a residue without a hydrogen atom of a hydroxyl group of lysolecithin having the hydroxyl group at the 2-position of glycerol and having formula (II)

- O-CH (CH₂OR) [CH₂OP (O) CO⁻) (OCH₂CH₂N⁺ (CH₃)₃] (II)

wherein R is a residue of fatty acid; m is an average number of bonds of lysolecithin to one molecule of superoxide dismutase which is a positive number of 1 or more. The present invention also provides a lyophilized drug composition for the treatment of motor neuron disease or ulcerative gastrointestinal injury. The present invention further relates to a lyophilized drug composition for the manufacture of a medicament for treating motor neuron disease or ulcerative gastrointestinal injury.
The drug composition of the present invention has preferably the following characteristics:
(a) property: when water for injection (distilled water for injection) is added to the lyophilized drug composition, it is dissolved and no insoluble foreign substances are observed;
(b) stability: when the superoxide dismutase activity per unit weight immediately after lyophilizing this drug composition in set as 100, the relative values of the activity at the time points after the lyophilized composition has been stored at 8°C for 12 months, 25°C for 12 months, or 40°C for 6 months are all 97% or more;
(c) peaks of analogues in gel filtration chromatography: when the lyophilized composition is re-dissolved, submitted to gel filtration chromatography and absorbance of the eluates is measured at 220 nm, no substantial difference is observed between the peak shape of lecithin-modified superoxide dismutase on the detection chart of the absorbance and the peak shape of lecithin-modified superoxide dismutase before lyophilized; and
(d) peaks of analogues by reversed phase chromatography: when the lyophilized composition was re-dissolved at the time points after it has been stored at 8°C for 12 months, 25°C for 12 months, or 40°C for 6 months and submitted to reversed phase chromatography and absorbance of the eluates is measured at 220 nm and 270 nm, each amount of detected analogues is not substantially different from that immediately after lyophilized.

The term "dissolution" in this specification means that a solute (lyophilized drug composition) and a solvent (water for injection) are mixed to generate a macroscopically clear solution. For whether "dissolution" occurs or not, it is preferable to judge whether or not the solute can be dissolved within 10 seconds by adding the solvent (water for injection) to the solute at room temperature (1°C to 30°C) and shaking mildly.

Further, the drug composition of the present invention preferably maintains all characteristics described in the above described (a) to (d) even at any time points after the lyophilized substance has been stored at 8°C for 36 months, 25°C for 36 months or 40°C for 6 months.

In addition, includes an agent having sucrose as an active ingredient is included for inhibiting (hereinafter, sometimes referred to as the inhibitor of the present invention) a reduction of the PC-SOD activity by making it coexist with PC-SOD represented by the above general formula (I), or for controlling appearances of peaks of analogues when analyzing the PC-SOD by column chromatography.

According to the present invention the reduction of the PC-SOD activity is inhibited by making sucrose coexist with PC-SOD represented by the above general formula (I), or the appearances of peaks of analogues when analyzing the PC-SOD by column chromatography is inhibited.

The drug composition of the present invention is useful as a drug, since reduction of the PCD-SOD activity due to long term storage is less (stability is higher) by an action of sucrose as a drug carrier, in the composition, the property when lyophilized is good and appearances of peaks of analogues when analyzed by column chromatography are controlled.

The drug composition of the present invention is used in the treatment of for diseases caused by excess active oxygen, in which a reduction of the PCD-SOD activity due to long term storage is less (stability is higher), the property when lyophilized is good and appearances of peaks of analogues when analyzed by column chromatography are less.

In addition, the present inventive inhibitor is useful when preparing the drug composition of the present invention, since it inhibits a reduction of the PCD-SOD activity due to long term storage (maintains stability), retains the property well when lyophilized and has an action of controlling appearances of peaks of analogues when analyzed by column chromatography.

### Best Mode for Carrying Out the Invention

The Embodiment will be described below.

### <1> The Present Inventive Drug Composition

### (1) PC-SOD used for the drug composition of the present invention

The term "lecithin" in the present specification designates normal lecithin which means phosphatidylcholine, and "lysolicithin" designates a compound in which one molecule of fatty acid bonded at the 2-position of glycerol in lecithin is removed and a hydroxyl group is bonded to the carbon atom at the 2-position.

The PC-SOD contained in the drug composition of the present invention dan be obtained usually by bonding one or more lecithin derivatives in which a chemical crosslinking agent is bonded to the hydroxyl group at the 2-position of lysolecithin to SOD. The PC-SOD is represented by the following formula (I):

SOD'(Q - B)ₘ (I)

In the above described general formula (I), SOD' is a residue of superoxide dismutase.

As far as SOD' in the above general formula (I) can exhibit the essential function of decomposing active oxygen O₂⁻ in vivo, the origin and the like are not particularly limited to and SOD residues originated in different animals and plants or microorganisms may be widely used. However, considering that it is preferable to reduce antigenicity as much as possible in a living body as SOD for use of drugs, it is preferable to select suitably appropriate SOD according to animal types to which the drug composition of the present invention is administered. For example, when targeting humans which may most need the drug composition of the present invention, it is preferable to use human-derived SOD so as to reduce antigenicity as SOD in a human living body as much as possible. Among them, human-derived Cu/Zn SOD (human-derived SOD containing copper and zinc at the center of activity; hereinafter, sometimes abbreviated as human Cu/Zn SOD) is especially preferable because there is much amount of expression in cells, a production technique by a gene engineering method is established and the much amount of preparation is possible.

This human Cu/Zn SOD includes: natural human Cu/Zn SOD manufactured from human tissues; human Cu/Zn SOD manufactured by a gene engineering method; recombinant human Cu/Zn SOD having substantially the same amino acid sequence as natural human Cu/Zn SOD; and SODs where partial amino acids in amino acid sequences of these human Cu/Zn SODs are modified or changed, and any human Cu/Zn SOD may be used.

For reference, the amino acid sequence of this natural human Cu/Zn SOD is shown in sequence No. 1. In addition, the actual human Cu/Zn SOD is constituted as a dimer of protein having this amino acid sequence.

While as shown in the amino acid sequence of this protein, the amino acid at the 111-position in natural human Cu/Zn SOD is cysteine, there are also reported human Cu/Zn SOD where conversion to serine was performed at the 111-position by a protein engineering method, e.g. a site-directed mutagenesis (Japanese Patent Laid-Open No. 62-130684), and human Cu/Zn SOD where cysteine at the 111-position was chemically modified (Japanese Patent Laid-Open No. 6-199895), and the PC-SOD of the present invention can be obtained using these human Cu/Zn SODs as a raw material. Among these human Cu/Zn SODs, the PC-SOD of the present invention is preferably obtained using the human Cu/Zn SOD as a raw material where cysteine at the 111-position was chemically modified, e.g. the cysteine was modified to S-(2-hydroxyethylthiocystein) which is uniform in the electrical charge and molecular weight, stable for the SOD activity.

Thus, in the present specification, both those in which partial amino acids are substituted in the human Cu/Zn SOD by a site-directed mutagenesis etc. and those obtained by chemically modifying partial amino acids of the human Cu/Zn SOD are simply designated as the human Cu/Zn SOD.

In addition, "a residue without a hydrogen atom of the hydroxyl group of lysolecithin having a hydroxyl group at the 2-position of glycerol" designated as B in the above described formula (I) is represented by the following formula (II):

-O-CH(CH₂OR)[CH₂OP(O)(O⁻)(OCH₂CH₂N*(CH₃)₃)] (II)

(wherein R is a residue of fatty acid (acyl group))

R is preferably a residue of saturated or unsaturated fatty acids of 10-28 carbons, more preferably a myristoyl group, a palmitoyl group, a stearoyl group, an icosanoyl group, a docosanoyl group, and other residues of saturated fatty acids of 14-22 carbons, most preferably a palmitoyl group which is a residue of saturated fatty acid of 16 carbons.

Q in the above described formula (I) represents chemical crosslinking. The chemical crosslinking is not particularly limited if it can be chemically bonded (covalent bond) crosslinking SOD with lecithin. For the chemical crosslinking, residue-C(O)-(CH₂)ₙ-C(O)- is preferably exemplified. This residue is preferably a residue without hydroxyl groups at the both ends of the straight-chained dicarboxylic acid represented by formula HO-C(O)-(CH₂)ₙ-C(O)-OH, or a residue without parts corresponding to hydroxyl groups at the both ends of the dicarboxylic acid anhydride, the dicarboxylic acid ester, the dicarboxylic acid halide, or other reactive derivatives and the like of the dicarboxylic acid. When Q in the above described formula (I) is a residue of the above described straight-chained dicarboxylic acid, one end of Q and B are linked through an ester bond. In such a case, the other end of the Q is considered to be directly bonded by an amide coupling etc. with an amino group of SOD. In such a case, SOD' shows a residue without one hydrogen atom of an amino group involved in the SOD bond. In the above described chemical crosslinking, n in group-(CH₂)ₙ- is an integer of 2 or more, preferably an integer of 2 to 10, most preferably 3.

m in the above described formula (I) represents the average number of bonds of lysolecithin to one molecule of SOD. m is one or more positive numbers, preferably 1-12 positive numbers, most preferably 4.

In manufacture of PC-SOD, coupling of a lecithin derivative to SOD can be performed according to the known method, e.g. the method described in Japanese Patent Laid-Open No. 6-54681. The manufacturing process of this PC-SOD is described in Examples.

It is preferable that the PC-SOD used in the drug composition of the present invention is purified to the extent that it is usable as a drug and substantially contains no substances which are not permitted as a drug.

For example, it is preferable to use the purified the PC-SOD having 2,500 U/mg (PC-SOD) or more specific activity, the purified one having 3,500 U/mg (PC-SOD) or more specific activity is more preferable. Herein, 1 U (unit) in the present specification means an enzymatic amount of the PC-SOD inhibiting 50% of the reduction rate of NBT (nitro blue tetrazolium) measured by the method according to J. Biol. Chem. Vol. 244, No. 22, 6049-6055 (1969) using NBT under the condition of pH 7.8 at 30°C.

### (2) Drug carrier used in the drug composition of the present invention

The drug composition fulfils all properties described in the aforementioned (a) to (d) with sucrose as regards solubility and stability. It is preferable that sucrose used in the drug composition of the present invention is purified to the extent that it is usable as a drug, substantially contains no substances which are not permitted as a drug, and treated with activated charcoal. Treatment with activated charcoal can be performed by dissolving sucrose in water followed by bringing this aqueous solution into contact with activated charcoal. Such an activated charcoal treatment, for example, can reduce an endotoxin concentration in sucrose, further can inhibit appearances of peaks of analogues and can improve stability.

The endotoxin concentration in the sucrose carrier used in the drug composition of the present invention is preferably 0.25 EU/mg or below, more preferably the detectable limit (0.006 EU/ml) or below.

While the endotoxin concentration, in the sucrose drug carrier can be measured using the known measuring method of endotoxin, the Limulus test method using limulina/amebocite/lysate components is preferable. As the Limulus reagent used in the Limulus test method, the endotoxin-specific Limulus reagent is preferably used. As the Limulus reagent, for example, the following commercially available Limulus reagents can be used; Toxicolor System LS-6, LS-20, LS-50M, Endospecy ES-6 and Endospecy ES-200 (Seikagaku Corporation).

### (3) The drug composition of the present invention

The drug composition of the present invention comprises PC-SOD and sucrose as a drug carrier. The composition is obtained by formulating the PC-SOD described in (1) and sucrose as a drug carrier and lyophilizing the composition described in (2). Thus, the drug composition of the present invention wherein: reduction of the PC-SOD activity due to long term storage is less (stability is higher); the property when lyophilized is excellent; and appearances of peaks of analogues when analyzed by column chromatography are controlled, can be obtained.

A mixing ratio of the PC-SOD to sucrose as a drug carrier in the drug composition of the present invention can be suitably determined by a person skilled in the art according to the dosage mount, the form or the like of the drug composition of the present invention, and the weight ratio of "PC-SOD/ sucrose as a drug carrier is preferably about 0.1/100-80/100, more preferably 0.4/100-60/100, but not particularly limited to.

The fatty acid content in the drug composition of the present invention is preferably 0.13-0.15 µmol/mg protein. The fatty acid content can be measured using, for example, YMC Co., Ltd.-made "a kit for analyzing long chained- or short chained-fatty acids".

As far as the enzymatic activity of PC-SOD and the effect of the present invention are not affected, components used usually for drugs such as other active ingredients of drugs, a conventional excipient, a binder, a lubricant, a coloring agent, adisintegrator, abufferingagent, an isotonizing agent, a preservative and a soothing agent can be added to the drug composition of the present invention. For such components, substances which are physiologically acceptable, have purity to the extent that they are usable as a drug and substantially do not contain substances which are not permitted as a drug can be used.

Manufacture of the drug composition of the present invention can be performed using the PC-SOD and sucrose as a drug carrier and lyophilizing the composition with the pharmaceutically known methods.

The form of the drug composition of the present invention is a lyophilized form. That is, the drug composition of the present invention is the lyophilized drug composition.

Lyophilizing can be performed through suitably setting conditions etc. by a person skilled in the art according to the known lyophilizing method. For example, using a commercially available lyophilizer, this can be performed by setting so as to be carried out in order of the following steps (1) to (5). Herein, numbers in the parentheses shows time required for each step.
(1) Cooling: for example, cool from a room temperature to -40°C to -50°C (about 0.3 to 1.5 hr).
(2) Maintaining the cooling state while keeping a vacuum state: for example, maintaining -40°C to -50°C (12 hr or more) .
(3) Returning to a room temperature from the cooling state while keeping the vacuum state: for example, returning to a room temperature from -40°C to -50°C (about 3 to 15 hr).
(4) Maintaining the room temperature while keeping the vacuum state (e.g. about 3 to 20 hr).
(5) Returning to normal pressure.

In manufacture of the lyophilized drug composition composed of steps (1) to (5), time required for the above step (2) should be preferably devised and the time is preferably 12 hrs or more.

For the lyophilized drug composition, the one which characteristics fulfill all conditions of the following (a)-(d) is particularly preferable.
(a) Property: when the lyophilized drug composition is observed macroscopically, the cake form of that is a tablet shape, and when water for injection is added to the lyophilized drug composition, it is rapidly dissolved and no insoluble foreign substances are observed (the property of the lyophilized drug composition is maintained well). Usually, it is dissolved in water for injection within 10 seconds.
(b) Stability: when the enzymatic activity per unit weight immediately after lyophilizing is set as 100 (%), the relative values (%) of the enzymatic activity at the time points after the lyophilized composition has been stored at 8°C for 12 months , 25°C for 12 months or 40°C for 6 months are 97% or more, preferably 99% or more. This stability can be measured by the method described in "(1-2-2) Stability test" in Example 1 explained later.
(c) Peaks of analogues by gel filtration chromatography: when absorbance is measured at 220 nm, no substantial difference is observed between the peak shape of PC-SOD (in the lyophilized drug composition) in the detection chart of the absorbance and the peak shape of PC-SOD before lyophilizing. The chromatography is preferably carried out by the method described in "(1-2-3-1) Detection test for peaks of analogues by gel filtration chromatography" in Example 1 explained later.
(d) Peaks of analogues by reversed phase chromatography: after the lyophilized composition has been stored at 8°C for 12 months, 25°C for 12 months or 40°C for 6 months, the amount of analogues detected by measurement of absorbance at 220 nm and 270 nm is not changed substantially compared to that immediately after lyophilizing. The chromatography is preferably carried out by the method described in "(1-2-3-2) Detection test of peaks of analogues by reversed phase chromatography" in Example 1 explained later.

Further, the drug composition of the present invention preferably maintains all properties described in the above (a)-(d) also at any time points during the lyophilized composition has been stored at 8°C for 36 months, 25°C for 36 months or 40°C for 6 months.

The analogues described in the present invention comprise various substances generated by cleavage at various sites in mainly lecithin including liberated fatty acids etc. These analogues are considered to be generated by cleavage of lecithin linked to PC-SOD, thus, when much amount of lecithin linked to PC-SOD is cleaved, the function as PC-SOD is reduced, and when much amount of such analogues are contained, they are not preferable managerially for the quality, the standards, etc. as the drug.

While the drug composition of the present invention can be used as the final dosage form as it is for administration as a drug, it can be used as a raw material for a drug having other dosage forms, for example, a liquid preparation, a lyophilized preparation, etc.

The drug composition of the present invention is preferably used as a preparation for injection having PC-SOD as an active ingredient. For example, when providing the drug composition of the present invention as a preparation for injection of a solution state, the drug composition of the present invention of the solution state manufactured by the above described method can be filled up into an appropriate container such as an ampoule, a vial, and a syringe for injection and sealed, then it can be distributed itself or stored to be provided for administration as an injection.

When providing the drug composition of the present invention as a preparation for injection in a frozen state, the drug composition of the present invention in a freezing state can be retained in an appropriate container such as an ampoule, a vial, and a syringe for injection, etc. in a sealed state, then distributed or stored to be provided for administration as an injection by thawing before administration.

When providing the lyophilized drug composition of the present invention as a preparation for injection, the lyophilized drug composition of the present invention as a preparation for injection can be retained in an appropriate container such as an ampoule, a vial, and a syringe for injection, etc. in a sealed state, then distributed or stored to be provided for administration as an injection by dissolving it with distilled water for injection before administration. The drug composition of the present invention in the lyophilized state may be provided as a set with a solvent for dissolution.

The drug composition of the present invention is a form of the lyophilized composition for injection.

### <2> The Treatment Agent

The treatment agent is a treatment agent for diseases comprising the drug composition of the present invention. Further, the treatment agent includes a concept such as a therapeutic agent, a preventive agent, an inhibitor for deterioration, an improving agent, etc. for different diseases. For PC-SOD, sucrose, these blending amount, other components which may be contained, a dosage form, etc. used as constitutive components of the treatment agent the same modes as the above described drug composition of the present invention can be adopted.

Diseases to which the treatment agent is applied are not particularly limited to, as far as the diseases can be treated (treatment, prevention, maintenance (inhibition for deterioration), reduction (improvement of symptoms) and the like) by the action of PC-SOD.

The diseases treatable by the action of PC-SOD include those caused by excess reactive oxygen, more specifically include: motor neuron diseases (e.g. amyotrophic lateral sclerosis (ALS), spinal progressive muscle atrophy, familial spastic paraplegia, Charcot-Marie-leg, progressive bulbar palsy, juvenile-lateral upper limb muscle atrophy (Hirayama disease), etc.), ischemic reperfusion disorder, acute renal failures (prerenal acute renal failure, renal acute renal failure, postrenal acute renal failure, etc.), lupus nephritis (normal glomerular lupus nephritis, lupus nephritis accompanying mesangial proliferative change, focal segmental glomerulonephritis, diffuse proliferative glomerulonephritis, diffuse membraneous glomerulonephritis, terminal sclerosing glomerulonephritis, etc.), functional disorders (allolalia, motor function disorder by paralysis, visual disorder, hearing disorder, smell disturbance, sensory disturbance such as dysgeusia, mental deterioration, etc.) accompanying cerebrovascular disorder (encephalorrhagy, hypertensive cerebral hemorrhage, cerebral infarction, cerebral thorombosis, brain embolism, transient ischemic attack, hypertensive encephalopathy, etc.), interstitial pneumonia, fibrosis (lung fibrosis etc.), ulcerative gastrointestinal injuries (ulcerative colitis, Crohn's disease, etc.), arthritis, heat injury, etc. Among them, motor neuron diseases (particularly ALS) and ulcerative gastrointestinal injuries (ulcerative colitis and Crohn's disease) are preferable indications. In addition, fibrosis (particularly lung fibrosis) and interstitial pneumonia are also preferable indications.

The treatment agent has an improving action to these diseases and to symptoms accompanying these diseases by an action of PC-SOD contained. Herein, the listed disease names are exemplified, but the application scope of the treatment agent is not limited to such exemplified diseases.

It is preferable that the dosage forms described later are appropriately selected as those of the treatment agent according to the properties and progress stages of targeted diseases.

The treatment agent can be administered orally or parenterally depending on administration methods such as injection (intramuscularily, subcutaneously, intracutaneously, intravenously, etc.), peroral use, inhalation, etc. and can be appropriately formulated according to the administration methods. As a dosage form, while there are an injection (solution, suspension, emulsion, solid medicine for dissolution at use, etc.), a tablet, a capsule, a granule, a powder, a solution, a lipoid agent, gel, external powder, spray, inhalation powder, suppository, etc., the injection is preferable, and it is more preferable to prepare an injection for intravenous administration.

An amount of the active ingredient (PC-SOD) in the treatment agent and an administration amount of the above described treatment agent are varied depending on a preparation method of the formulation, a dosage form, a disease type, an administration method, an administration form, purpose for use of the above described treatment agent, a specific symptom of a patient (disease degree), body weight of a patient, etc., but not particularly limited to, for example, 0.5-100 mg (1,500-300,000 U) a day per adult can be exemplified as the clinical amount. In addition, for the dosing intervals of the above formulation, about once a day is possible and administration divided into 3 or 4 times is also possible.

While the treatment agent can be also prepared as the one administered to vertebrate animals capable of developing the above described diseases, particularly mammals, it is preferable to prepare the treatment agent for humans.

### <3> The Inhibitor and the Inhibitory Method

As described above, by making sucrose coexist with PC-SOD, reduction of the PC-SOD activity can be inhibited or appearances of peaks of analogues when analyzing the PC-SOD by column chromatography can be controlled.

Accordingly, the inhibitor is an agent having sucrose as an effective ingredient to inhibit reduction of the PC-SOD activity or to control appearances of peaks of analogues when analyzing the PC-SOD by column chromatography by making sucrose coexist with PC-SOD represented by the above described general formula (I). Preferably, the agent is used for both purposes of inhibiting reduction of the above described PC-SOD activity is inhibited and of controlling appearances of peaks of analogues when analyzing the above described PC-SOD by column chromatography.

The inhibitor further exhibits an effect of retaining the property of the lyophilized drug composition well in addition to the above inhibitory effects by being made to coexist with PC-SOD when preparing the lyophilized drug composition. Thus, the inhibitor is preferably used by being made to coexist with the above described PC-SOD particularly when preparing the lyophilized drug composition containing the above described PC-SOD.

Further, the inhibitory method is the one by which reduction of the PC-SOD activity is inhibited or appearances of peaks of analogues when analyzing the PC-SOD by column chromatography are controlled by making sucrose coexist with PC-SOD represented by the above described general formula (I). Preferably, the inhibitory method is used for both purposes of inhibiting reduction of the above described PC-SOD activity and of controlling appearances of peaks of analogues when analyzing the above described PC-SOD by column chromatography.

Sucrose used as an active ingredient of the inhibitor or used in the inhibitory method and its amount added into PC-SOD etc. are similar to those described in the above described <1>.

Further, inhibition of reduction of the PC-SOD activity, control of peaks of analogues when analyzing by column chromatography and good maintenance of the property of the lyophilized drug composition when used for preparation of the composition can be confirmed by the methods described in Examples explained later.

### Examples

The present invention will be specifically described by the following Manufacturing Examples and Examples. However, the present inventive technical scope should not be limited to these.

### [Manufacturing Example]

### <1> Preparation of Human Cu/Zn SOD

The SOD used is the one which was prepared according to the method described in Japanese Patent Laid-Open No. 6-199895. The amino acid at the 111-position of this human Cu/Zn SOD is S-(2-hydroxyethylthio) cysteine.

### <2> Preparation of PC-SOD

PC-SOD was prepared by preparing 2-(4-hydroxycarbonylbutyroyl) lysolecithin according to the method described in Japanese Patent Laid-Open No. 6-54681 followed by reacting this with the SOD of the above described <1>. After purification/concentration, the number of bonds of the lecithin derivative per molecule of SOD was obtained by analyzing the protein concentration in according to the Lowry method (Lowry, O. H. et al., J. Biol. Chem., vol. 193, 265 (1951)) and by analyzing a residual amino group of SOD in according to the TNBS method (trinitrobenzene sulfonate, Goodwin, J. F. et al., Clin. Chem. vol. 16, 24 (1970)), to find that the average number of bonds was 4.0. The aqueous solution of this PC-SOD showed blue-green to green and pH showed 7-8. In addition, when examining the molecular weight by SDS-polyacrylamide gel electrophoresis, it was about 18,000 per monomer of the PC-SOD subunit (PC-SOD is a homodimer of the subunit).

### [Example 1]

"PEG 4000" used in the present Example means polyethylene glycol (average molecular weight 4000).

### (1) The methods used in this Example will be described together as follows.

### (1-1) Lyophilizing

A solution composition was lyophilized using the lyophilizer (Model No. RL402BS; Kyowa Vacuum-made). The program of lyophilizing was set as performed in order of steps (1) to (5) in the following Table 1.

**Table 1**

| Step | Pressure | Temperature | Time Required |
|---|---|---|---|
| (1) | Cooling | 25°C → -40°C | 0.5 hours |
| | | | |
| (2) | Maintaining vacuum state | maintaining at -40°C at | 12 hours |
| | | | |
| (3) | Maintaining vacuum state | -40°C → 25°C | 12 hours |
| | | | |
| (4) | Maintaining vacuum state | maintaining at 25°C | 16.5 hours |
| | | | |
| (5) | Returning pressure by nitrogen gas | maintaining at 25°C | several seconds |

### (1-2) Different test methods

### (1-2-1) Property test

For the lyophilized composition, the property of the composition was examined by the following method.

Those where the cake form of the lyophilized composition was macroscopically tablet-like, and when added water for injection to the lyophilized composition, it dissolved within 10 seconds and there were no insoluble foreign substances observed were evaluated as ";", while those where the cake form of the lyophilized composition was not tablet-like or when added water for injection to the lyophilized composition, it did not dissolve within 10 seconds or there were insoluble foreign substances observed were evaluated as "x".

### (1-2-2) Stability test

For the lyophilized composition, the enzymatic activity of PC-SOD in the composition was measured by the following method.

Using a solution obtained by adding water for injection to the lyophilized composition for reconstitution and using NBT under the condition of pH 7.8 at 30°C, the activity was measured by the method according to J. Biol. Chem. Vol. 244, No. 22, 6049-6055 (1969) to obtain the enzymatic amount inhibiting 50% of the reduction rate of NBT as 1 U. Compared to the enzymatic activity immediately after lyophilizing set as 100 (%), the relative value (%) of the enzymatic activity after storage was calculated.

### (1-2-3) Detection test for peaks of analogues by column chromatography

### (1-2-3-1) Detection test for peaks of analogues by gel filtration chromatography (hereinafter, sometimes abbreviated as "GPC")

A solution obtained by adding water for injection to the lyophilized composition for re-dissolution was used as a sample, which was submitted to high performance liquid chromatography using the following column and mobile phase:
Column: YMC-Pack Diol-200 (YMC Co., Ltd.-made), and
Mobile phase: 45% acetonitrile/0.01M phosphate buffer.

The absorbance of the eluates at 220 nm was continuously detected to obtain the detection chart. The cases where no substantial difference was observed between peak shapes of PC-SOD on the detection chart and the peak shapes of the PC-SOD before lyophilizing were evaluated as ";", while the cases where the peaks not seen in those of PC-SOD before lyophilizing were observed were evaluated as "x".

### (1-2-3-2) Detection test of peaks of analogues by reversed phase chromatography (hereinafter, sometimes abbreviated as "CN")

The sample was prepared similarly to the above (1-2-3-1) and submitted to high performance liquid chromatography using the following column and mobile phase:
Column: cyanopropylated silica gel (YMC Co., Ltd.-made), and
Mobile phase: acetonitrile/0.1% trifluoroacetic acid (elution at concentration gradient of 20%-90% of acetonitrile).

The absorbance of the eluates at 220 nm and 270 nm was continuously detected. The total area (SI) of a peak group (peak group of analogues) appeared in the front of a PC-SOD peak (main peak) and the total area (ST) of the PC-SOD peak and SI were measured to calculate an amount (SI/ST; %) of analogues for each sample.

For Tables 7 and 8 described later, the relative values are shown when an amount of analogues immediately after lyophilizing in prescription 1 set as 1 for easy comparison.

### (2) Test for PC-SOD-containing drug composition

### (2-1) Influence 1 by types of ingredients in the composition

PC-SOD (0.4 mg/vial) manufactured in the above described manufacturing Example and different ingredients (numbers in parentheses in the Table show weight per vial) were dissolved in water for injection to manufacture the solution composition and subsequently this was lyophilized.

The lyophilized substance was submitted to the above described different tests and the results are shown in Table 2.

Those where even one of x was present in the evaluation items were evaluated as "x" , while those where no x was present in the evaluation items at all were evaluated as "T".

**Table 2**

| Ingredient | Property Test | GPC | Judgement |
|---|---|---|---|
| Alanine (20 mg) | ; | x | x |
| Inositol (20 mg) | ; | x | x |
| Mannitol (20 mg) | ; | x | x |
| Sorbitol (20 mg) | x | ; | x |
| Sucrose (20 mg) | ; | ; | T |
| Creatinine (10 mg) | x | x | x |
| Glycine (20 mg) | ; | x | x |
| PEG4000 (0.5 mg) | ; | x | x |
| Urea (20 mg) | ; | x | x |
| None | x | x | x |

From the results of Table 2, it was clarified that the property of the lyophilized drug composition became extremely good and appearances of peaks of analogues when analyzing by column chromatography (GPC) were controlled by formulating PC-SOD together with sucrose.

### (2-2) Influence 1 by types of ingredients

PC-SOD manufactured in the above described manufacturing Example and different ingredients shown in Table 3 were dissolved in water for injection to manufacture the solution composition, subsequently 0.5 mL each was separately poured into a vial and this was lyophilized according to the above described method.

The weight in the Table shows the one contained in a vial and "-" means no addition.

**Table 3**

| | PC-SOD | Sucrose | PEG4000 | Sorbitol | Mannitol |
|---|---|---|---|---|---|
| Prescription 1 | 2 mg | 100 mg | - | - | - |
| | | | | | |
| Prescription 2 | 2 mg | 5 mg | 10 mg | - | - |
| | | | | | |
| Prescription 3 | 2 mg | - | - | 5 mg | 10 mg |
| | | | | | |
| Prescription 4 | 2 mg | - | 10 mg | 5 mg | - |

The lyophilized drug composition of the above described prescription 1 to 4 was submitted to the property test, stability test and the detection test for peaks of analogues by gel filtration chromatography, and the detection test for peaks of analogues by reversed phase chromatography at the following time points (a) to (d):
(a) immediately after lyophilized (not stored),
(b) the time point stored at 40°C for 3 months after lyophilized,
(c) the time point stored at 25°C for 3 months after lyophilized, and
(d) the time point stored at 8°C for 3 months after lyophilized (the time point stored for 6 months in the prescription 1).

The results of each test are shown in Tables 4 to 7.

### (1) Property test

**Table 4**

| Prescription | (a) Immediately after lyophilized | (b) 40°C for months | (c) 3 25°C for 3 months | (d) 8°C for 3 (6) months |
|---|---|---|---|---|
| 1 | ; | ; | ; | ; |
| 2 | ; | ; | ; | ; |
| 3 | ; | ; | ; | ; |
| 4 | ; | ; | ; | ; |

Any prescription showed the good property.

### (2) Stability test

**Table 5**

| Prescription | (a) Immediately after lyophilized | (b) 40°C for months | (c) 3 25°C for 3 months | (d) 8°C for 3 (6) months |
|---|---|---|---|---|
| 1 | 100 | 100 | 110 | 102 |
| 2 | 100 | 97 | 114 | 110 |
| 3 | 100 | 114 | 103 | 102 |
| 4 | 100 | 99 | 94 | 91 |

The prescription containing sorbitol and polyethylene glycol (prescription 4) showed greater reduction of PC-SOD activity than that of other prescription.

### (3) Detection test for peaks of analogues by gel filtration chromatography

**Table 6**

| Prescription | (a) Immediately after lyophilized | (b) 40°C for 3 months | (c) 25°C for 3 months | (d) 8°C for 3 (6) months |
|---|---|---|---|---|
| 1 | ; | ; | ; | ; |
| 2 | ; | ; | ; | ; |
| 3 | ; | N.T. | N.T. | ; |
| 4 | ; | ; | ; | ; |

In Table 6, N. T. means no performance of test.

No peaks of analogues by gel filtration chromatography were detected in any prescription.

### (4) Detection test for peaks of analogues by reversed phase chromatography (220 nm)

**Table 7**

| Prescription | (a) Immediately after lyophilized | (b) 40°C for 3 months | (c) 25°C for 3 months | (d) 8°C for 3 (6) months |
|---|---|---|---|---|
| 1 | 1 | 1.0 | 1.0 | 1.1 |
| 2 | 1.5 | 1.8 | 1.6 | 1.8 |
| 3 | 1.5 | 3.4 | 2.2 | 1.8 |
| 4 | 1.4 | 3.4 | 2.2 | 1.8 |

In the prescriptioncontaining sorbitol and mannitol (prescription 3) and in the prescription containing sorbitol and polyethylene glycol(prescription 4), the peak area of analogues by reversed phase chromatography was about 1.4 to 3.4 times that of the prescription containing sucrose (prescription 1).

In addition, the experiments similar to the above prescription using 0.4 mg or 10 mg for the content of PC-SOD were conducted, consequently the similar results were obtained.

From the above results, it was found that the lyophilized composition containing PC-SOD and sucrose has extremely advantageous effects that: (1) the property is good; (2) reduction of enzymatic activity due to long-term storage is significantly less (extremely stable); and (3) peaks of analogues by gel filtration chromatography and reversed phase chromatography are extremely less, compared to the other prescription.

### [Example 2]

In order to examine whether or not the lyophilized composition containing PC-SOD and sucrose could resist against the longer-term storage, the following tests were performed.

The lyophilized composition containing PC-SOD was manufactured with the above prescription 1, subsequently different tests were conducted according to the same method as Example 1 at the following time points (e) to (h). For each prescription of the present tests, 3 lots were submitted to the tests, respectively:
(e) immediately after lyophilized (not stored),
(f) the time point when stored at 40°C for 6 months after lyophilized,
(g) the time point when stored at 25°C for 12 months (g-2), 24 months (g-2) and 36 months (g-3) after lyophilized, and
(h) the time point when stored at 8°C for 12 months (h-2), 24 months (h-2) and 36 months (h-3) after lyophilized.

The results of the tests are shown together in Table 8.

**Table 8**

| Test | (e) Immediately after lyophilized | (f) 40°C 6 months | (g-1) 25°C 12 months | (g-2) 25°C 24 months | (g-3) 25°C 36 months | (h-1) 8°C 12 months | (h-2) 8°C 24 months | (h-3) 8°C 36 months |
|---|---|---|---|---|---|---|---|---|
| Property | ; | ; | ; | ; | ; | ; | ; | ; |
| Stability | 100 | 102 | 106 | 111 | 112 | 105 | 110 | 105 |
| GPC | ; | ; | ; | ; | ; | ; | ; | ; |
| CN | 1.0 | 1.2 | 1.0 | 1.0 | 1.1 | 0.9 | 1.0 | 1.1 |
| | 1.0 | 1.3 | 1.0 | 0.6 | 1.1 | 0.9 | 0.8 | 1.1 |

| | | | | | | | | |
|---|---|---|---|---|---|---|---|---|
| (the upper row of the column CN shows the results of 220 nm and the lower row shows those of 270 nm) | | | | | | | | |

The experiments similar to this prescription using 0.4 mg or 10 mg for the content of PC-SOD were conducted, consequently the similar results are shown in Tables 9 and 10, respectively.

**Table 9**

| Test | (e) Immediately after lyophilized | (f) 40°C 6 months | (g-1) 25°C 12 months | (g-2) 25°C 24 months | (g-3) 25°C 36 months | (h-1) 8°C 12 months | (h-2) 8°C 24 months | (h-3) 8°C 36 months |
|---|---|---|---|---|---|---|---|---|
| Property | ; | ; | ; | ; | ; | ; | ; | ; |
| Stability | 100 | 104 | 104 | 110 | 104 | 104 | 110 | 108 |
| GPC | ; | ; | ; | ; | ; | ; | ; | ; |
| CN | 1.0 | 1.0 | 0.8 | 0.9 | 1.0 | 0.7 | 0.9 | 1.1 |
| | 1.0 | 1.6 | 0.9 | 0.7 | 1.0 | 0.8 | 0.7 | 1.1 |

| | | | | | | | | |
|---|---|---|---|---|---|---|---|---|
| (the upper row of the column CN shows the results of 220 nm and the lower row shows those of 270 nm) | | | | | | | | |

**Table 10**

| Test | (e) Immediately after lyophilized | (f) 40°C 6 months | (g-1) 25°C 12 months | (g-2) 25°C 24 months | (g-3) 25°C 36 months | (h-1) 8°C 12 months | (h-2) 8°C 24 months | (h-3) 8°C 36 months |
|---|---|---|---|---|---|---|---|---|
| Stability | 100 | 101 | 104 | 108 | 110 | 104 | 104 | 105 |
| GPC | ; | ; | ; | ; | ; | ; | ; | ; |
| CN | 1.0 | 1.2 | 1.0 | 1.1 | 1.2 | 1.1 | 1.0 | 1.1 |
| | 1.0 | 1.1 | 0.9 | 0.8 | 1.1 | 0.9 | 0.8 | 1.1 |

| | | | | | | | | |
|---|---|---|---|---|---|---|---|---|
| (the upper row of the column CN shows the results of 220 nm and the lower row shows those of 270 nm) | | | | | | | | |

### [Example 3]

30 mg of PC-SOD manufactured in the above described manufacturing Example and 50 mg of sucrose were dissolved in water for injection to manufacture the solution composition, subsequently 1.5 mL each was separately poured into a vial to lyophilize them according to the above described method.

At the time point immediately after lyophilizing the above described composition and that when stored at 8°C for 9 months, different tests were performed according to the same methods as in Example 1 except for the measuring method of the specific activity. The specific activity was measured by the method according to J. Biol. Chem., Vol. 244, No. 22, 6049-6055 (1969) using a solution which was obtained by adding water for injection to the lyophilized composition for re-dissolution and using cytochrome C under the condition of pH 7.8 at 25°C to obtain the enzymatic amount inhibiting 50% of the reduction rate of cytochrome C set as 1 U. Compared to the enzymatic activity immediately after lyophilizing was set as 100 (%), the relative values (%) of the enzymatic activity after storage were calculated. The results of tests are shown in Table 11.

**Table 11**

| Test | Immediately after lyophilized | 8°c 6 months | 8°C 9 months | 25°C 6 months |
|---|---|---|---|---|
| Property | ; | ; | ; | ; |
| Stability | 100 | 104 | 123 | 109 |
| GPC | ; | ; | ; | ; |
| CN | 1.0 | 1.2 | 1.0 | 1.2 |
| | 1.0 | 1.3 | 1.1 | 1.3 |

| | | | | |
|---|---|---|---|---|
| (the upper row of the column CN row shows the results of 220 nm and the lower row shows those of 270 nm) | | | | |

From the above results, it was demonstrated that sucrose exhibited effects of retaining the property of the lyophilized drug composition well even after long-term storage, inhibiting reduction of the PC-SOD activity (retaining stably), controlling peaks of analogues when analyzed by gel filtration chromatography and reversed phase chromatography etc. by being added to PC-SOD besides the known effect as the drug carrier (binder).

## Claims

1. A lyophilized drug composition **characterized by** including sucrose as a drug carrier and a lecithin-modified superoxide dismutase represented by the following general formula (I):
SOD' (Q - B)ₘ (I)
**characterized in that** SOD' is a residue of superoxide dismutase: Q is a chemical crosslinking; B is a residue without a hydrogen atom of a hydroxyl group of lysolecithin having the hydroxyl group at the 2-position of glycerol and having formula (II):
-O-CH (CH₂OR) [CH₂OP (O) (O⁻) (OCH₂CH₂N⁺ (CH₃)₃] (II)
wherein R is a residue of fatty acid; m is an average number of bonds of lysolecithin to one molecule of superoxide dismutase which is a positive number of 1 or more.

2. The drug composition according to claim 1 **characterized in that** a fatty acid content in the drug composition is 0.13-0.15 µmol/mg protein.

3. The drug composition according to claim 1 or 2, **characterized in that** Q is -C(O)-(CH₂)ₙ-C(O)- wherein n is an integer of 2 or more.

4. The drug composition according to any of claims 1 to 3 **characterized in that** SOD' is a residue of human superoxide dismutase.

5. The drug composition according to any of claims 1 to 4 **characterized in that** SOD' is a residue of a modified form of superoxide dismutase in which an amino acid in 111-position of an amino acid sequence of human superoxide dismutase is converted into S-(2-hydroxyethylthio) cysteine.

6. The drug composition according to claim 4 or 5 **characterized in that** the superoxide dismutase contains copper and zinc at the active center.

7. The drug composition according to any of claims 3 to 6 **characterized in that** n is an integer of 2 to 10.

8. The drug composition according to any of claims 1 to 7 **characterized in that** m is a positive number of 1 to 12.

9. The drug composition according to any of claims 1 to 8 **characterized in that** sucrose is treated with activated charcoal.

10. The drug composition according to any of claims 1 to 9 **characterized in that** a weight ratio of lecithinized superoxide dismutase to sucrose is 0.4/100-60/100.

11. A drug composition according to any of claims 1 to 10 for use in the treatment of motor neuron disease or ulcerative gastrointestinal injury.

12. Use of a drug composition according to any of claims 1 to 10 for the manufacture of a medicament for treating motor neuron disease or ulcerative gastrointestinal injury.

13. A method for producing the drug composition according to claim 1 by formulating sucrose as a carrier and a lecithin-modified superoxide dismutase represented by the general formula (I) and lyophilizing the composition.

## Patentansprüche

1. Lyophilisierte Medikamentenzusammensetzung, **dadurch gekennzeichnet, dass** sie Saccharose als Medikamententräger und eine lecithin-modifizierte Superoxid-Dismutase enthält, die durch die nachfolgende allgemeine Formel (I) repräsentiert ist:
SOD' (Q - B)ₘ (I)
**dadurch gekennzeichnet, dass** SOD' ein Rest von Superoxid-Dismutase ist; Q eine chemische Quervernetzung ist; B ein Rest ohne Wasserstoffatom einer Hydroxylgruppe von Lysolecithin mit der Hydroxylgruppe an der 2-Position von Glycerin ist und die Formel (II) hat:
-O-CH (CH₂OR) [CH₂OP (O) (O⁻) (OCH₂CH₂N⁺ (CH₃)₃] (II)
wobei R ein Rest Fettsäure ist; M eine Durchschnittsanzahl von Bindungen von Lysolecithin mit einem Molekül von Superoxid-Dismutase ist, die eine positive Zahl von 1 oder mehr ist.

2. Medikamentenzusammensetzung gemäß Anspruch 1, **dadurch gekennzeichnet, dass** ein Fettsäuregehalt in der Medikamentenzusammensetzung 0,13-0,15 µmol/mg Protein beträgt.

3. Medikamentenzusammensetzung gemäß Anspruch 1 oder 2, **dadurch gekennzeichnet, dass** Q = -C(O) - (CH₂)ₙ-C(O)- ist, wobei n eine Ganzzahl von 2 oder mehr ist.

4. Medikamentenzusammensetzung gemäß einem der Ansprüche 1 bis 3, **dadurch gekennzeichnet, dass** SOD' ein Rest von menschlicher Superoxid-Dismutase ist.

5. Medikamentenzusammensetzung gemäß einem der Ansprüche 1 bis 4, **dadurch gekennzeichnet, dass** SOD' ein Rest einer modifizierten Form von Superoxid-Dismutase ist, in der eine Aminosäure an der 111-Position einer Aminosäuresequenz menschlicher Superoxid-Dismutase zu S-(2-Hydroxyehtylthio)Cystein konvertiert ist.

6. Medikamentenzusammensetzung gemäß Anspruch 4 oder 5, **dadurch gekennzeichnet, dass** die Superoxid-Dismutase Kupfer und Zink im aktiven Zentrum enthält.

7. Medikamentenzusammensetzung gemäß einem der Ansprüche 3 bis 6, **dadurch gekennzeichnet, dass** n eine Ganzzahl von 2 bis 10 ist.

8. Medikamentenzusammensetzung gemäß einem der Ansprüche 1 bis 7, **dadurch gekennzeichnet, dass** m eine Positivzahl von 1 bis 12 ist.

9. Medikamentenzusammensetzung gemäß einem der Ansprüche 1 bis 8, **dadurch gekennzeichnet, dass** Saccharose mit Aktivkohle behandelt ist.

10. Medikamentenzusammensetzung gemäß einem der Ansprüche 1 bis 9, **dadurch gekennzeichnet, dass** ein Gewichtsverhältnis von lecithinisierter Superoxid-Dismutase zu Saccharose 0,4/100-60/100 ist.

11. Medikamentenzusammensetzung gemäß einem der Ansprüche 1 bis 10 zur Verwendung bei der Behandlung von motorischer Neuronenerkrankung oder ulcerativer Gastrointestinalverletzung.

12. Verwendung einer Medikamentenzusammensetzung gemäß einem der Ansprüche 1 bis 10 für die Herstellung eines Medikaments zur Behandlung einer motorischen Neuronenkrankheit oder ulcerativen Gastrointestinalverletzung.

13. Verfahren zum Herstellen einer Medikamentenzusammensetzung gemäß Anspruch 1 durch Formulieren von Saccharose als einem Träger und einer lecithinmodifizierten Superoxid-Dismutase, die durch die allgemeine Formel (I) repräsentiert ist, und Lyophilisieren der Zusammensetzung.

## Revendications

1. Composition médicamenteuse lyophilisée **caractérisée par** le fait d'inclure du sucrose comme support médicamenteux et une superoxyde dismutase lécithinisée représentée par la formule générale suivante (I) :
SOD' (Q - B)ₘ (I)
**caractérisée en ce que** la SOD' est un résidu de superoxyde dismutase, Q est une réticulation chimique, B est un résidu sans atome d'hydrogène d'un groupe hydroxyle de la lysolécithine dont le groupe hydroxyle se trouve à la position 2 du glycérol et qui a la formule (II) :
-O-CH(CH₂OR)[CH₂OP(O)(O⁻)(OCH₂CH₂N⁺(CH₃)₃] (II)
dans laquelle R est un résidu d'acide gras, m est un nombre moyen de liaisons de la lysolécithine à une molécule de superoxyde dismutase et qui est en nombre positif égal à 1 ou plus.

2. Composition médicamenteuse selon la revendication 1, **caractérisée en ce que** la teneur en acide gras dans la composition médicamenteuse est de 0,13 à 0,15 µmol/mg de protéine.

3. Composition médicamenteuse selon la revendication 1 ou 2, **caractérisée en ce que** Q est -C(O)-(CH₂)ₙ-C(O)- où n est un entier égal à 2 ou plus.

4. Composition médicamenteuse selon l'une quelconque des revendications 1 à 3, **caractérisée en ce que** la SOD' est un résidu de superoxyde dismutase humaine.

5. Composition médicamenteuse selon l'une quelconque des revendications 1 à 4, **caractérisée en ce que** la SOD' est un résidu d'une forme modifiée de superoxyde dismutase dans laquelle un acide aminé en position 111 d'une séquence aminoacide de superoxyde dismutase humaine est converti en S-(2-hydroxyéthylthio)cystéine.

6. Composition médicamenteuse selon la revendication 4 ou 5, **caractérisée en ce que** la superoxyde dismutase contient du cuivre et du zinc au niveau du centre actif.

7. Composition médicamenteuse selon l'une quelconque des revendications 3 à 6, **caractérisée en ce que** n est un entier allant de 2 à 10.

8. Composition médicamenteuse selon l'une quelconque des revendications 1 à 7, **caractérisée en ce que** m est un nombre positif allant de 1 à 12.

9. Composition médicamenteuse selon l'une quelconque des revendications 1 à 8, **caractérisée en ce que** le sucrose est traité avec du charbon activé.

10. Composition médicamenteuse selon l'une quelconque des revendications 1 à 9, **caractérisée en ce qu'**un rapport en poids entre la superoxyde dismutase lécithinisée et le sucrose est de 0,4/100 à 60/100.

11. Composition médicamenteuse selon l'une quelconque des revendications 1 à 10 pour une utilisation dans le traitement d'une maladie atteignant les neurones moteurs ou d'un ulcère gastro-intestinal.

12. Utilisation d'une composition médicamenteuse selon l'une quelconque des revendications 1 à 10 pour la fabrication d'un médicament pour le traitement d'une maladie atteignant les neurones moteurs ou d'un ulcère gastro-intestinal.

13. Procédé de fabrication de la composition médicamenteuse selon la revendication 1 en formulant le sucrose comme porteur et une superoxyde dismutase lécithinisée représentée par la formule générale (I) et en lyophilisant la composition.
